# EUROPEAN PATENT APPLICATION

(11) **EP 3 795 157 A1**
(43) Date of publication of application: **24.03.2021**
(21) Application number: 19803255.9
(22) Date of filing: 13.05.2019
(51) Int. Cl.: A61K 31/506, A61K 9/28, A61K 47/26, A61P 35/00

(54) **PHARMACEUTICAL COMPOSITION COMPRISING SMALL MOLECULE EGFR INHIBITOR AND PREPARATION METHOD THEREFOR**

(30) Priority: 15.05.2018 CN 201810463668
(71) Applicant: Jiangsu Hansoh Pharmaceutical Group Co., Ltd., Lianyungang, Jiangsu 222047 (CN)
(72) Inventor: CHEN, Weiqi, Lianyungang, Jiangsu 222047 (CN); ZHAO, Junjun, Lianyungang, Jiangsu 222047 (CN); WANG, Xiaolei, Lianyungang, Jiangsu 222047 (CN); YIN, Baoqing, Lianyungang, Jiangsu 222047 (CN); CHEN, Xiao, Lianyungang, Jiangsu 222047 (CN)
(74) Representative: Freischem & Partner Patentanwälte mbB
(86) International application number: PCT/CN2019/086569
(87) International publication number: WO 2019/218958

(57) **Abstract**

A pharmaceutical composition comprising a small molecule EGFR inhibitor and a preparation method therefor, the composition comprising N-(5-((4-(1-cyclopropyl-1H-indol-3-yl)pyrimidin-2-yl)amino)-2-((2-(dimethylamino)et hyl)(methyl)amino)-4-methoxyphenyl)acrylamide, an isomer, solvate, hydrate, or pharmaceutically acceptable salt thereof, or a combination thereof that acts as an active ingredient, and at least one pharmaceutically acceptable excipient.

## Description

### FIELD OF THE INVENTION

The present invention belongs to the field of pharmaceutical formulations, and specifically relates to a pharmaceutical composition comprising a third-generation small molecule EGFR inhibitor and a method for preparing the same.

### BACKGROUND OF THE INVENTION

EGFR (Epidermal Growth Factor Receptor) is a member of the erbB receptor family, which includes transmembrane protein tyrosine kinase receptors. By binding to its ligand, such as epidermal growth factor (EGF), EGFR can form a homodimer on the cell membrane or form a heterodimer with other receptors in the family, such as erbB2, erbB3, or erbB4. The formation of these dimers can cause the phosphorylation of key tyrosine residues in EGFR cells, thereby activating a number of downstream signaling pathways in cells. These intracellular signaling pathways play an important role in cell proliferation, survival and anti-apoptosis. Disorders of EGFR signal transduction pathways, including increased expression of ligands and receptors, EGFR gene amplification and mutation and the like, can promote malignant transformation of cells, and play an important role in tumor cell proliferation, invasion, metastasis and angiogenesis. Therefore, EGFR is a reasonable target for the development of anticancer drugs.

N-(5-((4-(1-cyclopropyl-1H-indol-3-yl)pyrimidin-2-yl)amino)-2-((2-(dimethylami no)ethyl)(methyl)amino)-4-methoxyphenyl)acrylamide, which belongs to the third-generation small molecule EGFR inhibitor, has high selectivity for inhibiting the EGFR T790M mutant, while has no or low activity on wild-type EGFR, therefore, it can be used in treating drug-resistant tumors caused by the secondary mutation of EGFR-T790M. This compound (hereinafter referred to as the compound of formula I) was first disclosed in the international patent application WO2016054987, and its structure is shown in the following formula I:

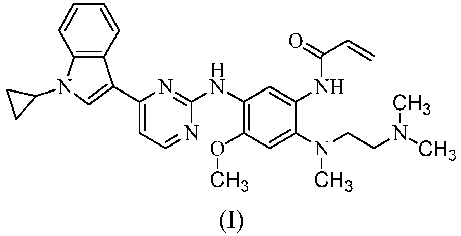

### SUMMARY OF THE INVENTION

The present invention provides a pharmaceutical composition comprising a third-generation small molecule EGFR inhibitor.

The pharmaceutical composition comprises a mesylate salt of N-(5-((4-(1-cyclopropyl-1H-indol-3-yl)pyrimidin-2-yl)amino)-2-((2-(dimethylamino)et hyl)(methyl)amino)-4-methoxyphenyl)acrylamide, a polymorph, solvate, hydrate, pharmaceutically acceptable salt thereof or a combination thereof as the active ingredient, and at least one pharmaceutically acceptable excipient.

In the pharmaceutical composition according to the present invention, the active ingredient is present in an amount of 1 to 60%.

In the pharmaceutical composition according to the present invention, the unit dose of the active ingredient is 10 to 200 mg, preferably 55 to 110 mg, and more preferably 55 mg or 110 mg.

In the pharmaceutical composition according to the present invention, the excipient comprises one or more filler(s), comprising at least one disaccharide or polysaccharide, such as glucan, starch, cellulose, lactose, maltose or sucrose. The starch can be selected from the group consisting of potato starch, corn starch, rice starch, wheat starch, amylopectin, pregelatinized starch and the like. The disaccharide or polysaccharide is present in an amount of 1 to 60%, preferably 5 to 55%, more preferably 5 to 30%, and further preferably 5 to 15%.

The filler is preferably a disaccharide, such as lactose, and it is present in an amount of 1 to 60%, preferably 5 to 55%, more preferably 5 to 30%, and further preferably 5 to 15%.

In the pharmaceutical composition according to the present invention, the excipient comprises one or more filler(s), disintegrant(s) or lubricant(s).

In the pharmaceutical composition according to the present invention, the weight percentage of each component is as follows:

| | |
|---|---|
| active ingredient | 1 to 60% |
| filler | 20 to 80% |
| disintegrant | 1 to 30% |
| lubricant | 0.1 to 10%. |

Preferably, the weight percentage of each component is as follows:

| | |
|---|---|
| active ingredient | 35 to 50% |
| filler | 30 to 50% |
| disintegrant | 10 to 20% |
| lubricant | 0.5 to 5%. |

More preferably, the weight percentage of each component is as follows:

| | |
|---|---|
| active ingredient | 43.3% |
| filler | 40.2% |
| disintegrant | 14% |
| lubricant | 2.5%. |

In the pharmaceutical composition according to the present invention, the filler comprises one or more of microcrystalline cellulose, lactose, anhydrous lactose, corn starch, pregelatinized starch, mannitol, sorbitol, calcium hydrophosphate and calcium sulfate, wherein the filler is present in an amount of 20 to 80%, and preferably 30 to 50%. Preferably, the filler comprises two of the above components, and the weight ratio of the two components is 1:3 to 3:1.

In the pharmaceutical composition according to the present invention, the filler is preferably microcrystalline cellulose and lactose. The microcrystalline cellulose is present in an amount of 1 to 60%, preferably 10 to 40%, and more preferably 20 to 40%, relative to the weight of the tablet core; and the lactose is present in an amount of 1 to 60%, preferably 5 to 30%, and more preferably 5 to 15%, relative to the weight of the tablet core. Optional technical solutions include, but are not limited to, the microcrystalline cellulose is present in an amount of 1 to 60%, and the lactose is present in an amount of 1 to 60%; the microcrystalline cellulose is present in an amount of 10 to 40%, and the lactose is present in an amount of 5 to 30%; and the microcrystalline cellulose is present in an amount of 20 to 40%, and the lactose is present in an amount of 5 to 15%.

Further preferably, the weight ratio of microcrystalline cellulose to lactose is 1:3 to 3:1, and more preferably 2-3:1.

Further preferably, the lactose is anhydrous lactose. The microcrystalline cellulose is present in an amount of 1 to 60%, preferably 10 to 40%, and more preferably 20 to 40%, relative to the weight of the tablet core; and the anhydrous lactose is present in an amount of 1 to 60%, preferably 5 to 30%, and more preferably 5 to 15%, relative to the weight of the tablet core. Optional technical solutions include, but are not limited to, the microcrystalline cellulose is present in an amount of 1 to 60%, and the anhydrous lactose is present in an amount of 1 to 60%; the microcrystalline cellulose is present in an amount of 10 to 40%, and the anhydrous lactose is present in an amount of 5 to 30%; and the microcrystalline cellulose is present in an amount of 20 to 40%, and the anhydrous lactose is present in an amount of 5 to 15%.

Further preferably, the weight ratio of microcrystalline cellulose to anhydrous lactose is 1:3 to 3:1, more preferably 2-3:1.

Through the addition of lactose, the pharmaceutical composition with a high content of the compound of formula I can be prepared, and its drug loading capacity can reach more than 40%. At the same time, the resulting pharmaceutical composition has a good compressibility and uniform dissolution.

In the pharmaceutical composition according to the present invention, the disintegrant comprises one or more of low-substituted hydroxypropyl cellulose, croscarmellose sodium, carboxymethyl starch sodium and crospovidone, and preferably carboxymethyl starch sodium. The disintegrant is present in an amount of 1 to 30%, and preferably 10 to 20%. The disintegrant that is added in a totally intragranular manner can ensure good disintegration and preparation of the tablet. The addition of carboxymethyl starch sodium can further improve the stability of the formulation. Preferably, the disintegrant is added intragranularly.

In the pharmaceutical composition according to the present invention, the lubricant comprises one or more of talc, stearic acid, sodium stearyl fumarate, glyceryl behenate, magnesium stearate and micronized silica gel. The lubricant is present in an amount of 0.1 to 10%, and preferably 0.2 to 5%, relative to the weight of the tablet core.

Further, the lubricant is preferably sodium stearyl fumarate and magnesium stearate. The sodium stearyl fumarate is present in an amount of 0.1 to 5%, and preferably 0.3 to 3%, and the magnesium stearate is present in an amount of 0.1 to 5%, and preferably 0.2 to 2%. The combination of sodium stearyl fumarate and magnesium stearate can obviously improve the adhesion of raw materials on the surface of the equipment, ensure a smooth preparation process, and enable the finished product to have an excellent dissolution effect.

In the pharmaceutical composition according to the present invention, the amount refers to the percentage relative to the total weight of the drug, wherein the total weight of the drug does not include the weight of the coating. As for specific dosage forms, the amount refers to the percentage relative to the weight of the tablet core or granule.

The pharmaceutical composition according to the present invention comprises the following components:

| | |
|---|---|
| active ingredient | 35 to 50% |
| lactose | 5 to 15% |
| microcrystalline cellulose | 30 to 50% |
| carboxymethyl starch sodium | 10 to 20% |
| lubricant | 0.5 to 5%. |

Preferably, it comprises the following components:

| | |
|---|---|
| active ingredient | 43.3% |
| lactose | 10.2% |
| microcrystalline cellulose | 30.0% |
| carboxymethyl starch sodium | 10.4% |
| lubricant | 0.5 to 5%. |

More preferably, it comprises the following components:

| | |
|---|---|
| active ingredient | 43.3% |
| lactose | 10.2% |
| microcrystalline cellulose | 30.0% |
| carboxymethyl starch sodium | 14% |
| lubricant | 2.5%. |

Most preferably, it comprises the following components:

| | |
|---|---|
| active ingredient | 43.3% |
| lactose | 10.2% |
| microcrystalline cellulose | 30.0% |
| carboxymethyl starch sodium | 14% |
| magnesium stearate | 1% |
| sodium stearyl fumarate | 1.5%. |

The pharmaceutical composition of the present invention is an oral formulation selected from the group consisting of tablet and capsule, preferably a film, and more preferably an immediate-release film-coated tablet. The coating agent is a gastric-soluble film coating premix, preferably Opadry 85F140124 (the main ingredients are polyvinyl alcohol, polyethylene glycol, talc, titanium dioxide, iron oxide red and iron oxide black), and Opadry 85F12300 (the main ingredients are polyvinyl alcohol, polyethylene glycol, talc, titanium dioxide and iron oxide yellow).

The present invention further provides a method for preparing the pharmaceutical composition, mainly comprising the following steps of:
1) pre-treatment of raw materials: sieving the filler, disintegrant and lubricant for later use;
2) mixing: weighing the intragranular raw materials according to specified amounts and mixing them;
3) dry granulation: granulating the above mixed powder by dry granulation;
4) total mixing: mixing the resulting granules and extragranular lubricant;
5) optionally, tableting: compressing the resulting mixture into tablets according to the theoretical tablet weight; and
6) optionally, coating.

The above method comprises the specific steps of:
1) pre-treatment of raw materials: sieving microcrystalline cellulose and anhydrous lactose with a 60-mesh sieve, and sieving sodium stearyl fumarate and carboxymethyl starch sodium with an 80-mesh sieve for later use;
2) mixing: weighing the intragranular raw materials according to prescription amounts, and mixing microcrystalline cellulose, anhydrous lactose, carboxymethyl starch sodium, sodium stearyl fumarate, the active ingredient and magnesium stearate with a hopper mixer;
3) dry granulation: granulating the above mixed powder with a dry granulator;
4) total mixing: mixing the resulting fine granules and prescription amount of extragranular sodium stearyl fumarate with a hopper mixer;
5) optionally, tableting: compressing the resulting mixture into tablets according to the theoretical tablet weight; and
6) optionally, coating: i) formulation of a coating liquid, adding a prescription amount of Opadry to purified water under stirring to formulate a coating liquid with a solid content of 10%, stirring the coating liquid for 60 minutes after the completion of the addition of Opadry to make it evenly dispersed, and sieving the formulated coating liquid with a 100-mesh sieve for later use; and ii) setting the parameters according to the process requirements, and finishing the coating until the coating weight gain reaches about 2.0% to 4.0%.

The inventor finds through a large number of studies that N-(5-((4-(1-cyclopropyl-1H-indol-3-yl)pyrimidin-2-yl)amino)-2-((2-(dimethylamino)et hyl)(methyl)amino)-4-methoxyphenyl)acrylamide, as a third-generation small molecule EGFR inhibitor, is a drug with low permeability. Its solubility is greatly affected by pH, which is pH dependent. The pharmaceutical composition prepared according to the present invention has a good and uniform dissolution in different dissolution media.

### DETAILED DESCRIPTION OF THE INVENTION

### Regarding to the filler:

It can be found from Table 1 that when the filler comprises lactose, a pharmaceutical composition with a high content of the compound of formula I can be prepared, and the resulting pharmaceutical composition has a good compressibility.

**Table 1 Screening of the filler**

| **Single-factor study** | **Sample 1** | **Sample 2** | **Sample 3** |
|---|---|---|---|
| **Active ingredient ¹** | 1.416 | 1.416 | 1.416 |
| **Microcrystalline cellulose** | 1.05 | 1.05 | 1.05 |
| **Anhydrous lactose** | 3.15 | / | 1.56 |
| **Mannitol** | / | 3.15 | 1.59 |
| **Croscarmellose sodium (added intragranularly)** | 0.18 | 0.18 | 0.18 |
| **Croscarmellose sodium (added extragranularly)** | 0.09 | 0.09 | 0.09 |
| **Magnesium stearate (added intragranularly)** | 0.09 | 0.09 | 0.09 |
| **Magnesium stearate (added extragranularly)** | 0.084 | 0.084 | 0.084 |
| **Process of compressing large tablets (called "slugs")** | There was no punch sticking, and the large tablets were smooth and 100% qualified | There was punch sticking, and the large tablets were not smooth and does not meet the production requirement | There was punch sticking, and the large tablets were not smooth and does not meet the production requirement |

| | | | |
|---|---|---|---|
| Note: The active ingredient is calculated by mesylate salt, the same below. | | | |

The drug loading capacity of the active ingredient in the tablet was further increased. When the active ingredient was present in an amount of 43.3%, the tablet still had a very good compressibility. There was no punch sticking. The resulting uncoated tablets were smooth and 100% qualified, having a complete shape and good appearance.

**Table 2 Type and dosage of the filler**

| **Single-factor study** | | | **Microcrystalline cellulose (PH102) + anhydrous lactose (21AN)** | | |
|---|---|---|---|---|---|
| | | | **Ratio: 3:1** | **Ratio: 1:1** | **Ratio: 1:3** |
| **Batch** | | | 200 Tablets/g | 200 Tablets/g | 200 Tablets/g |
| **Active ingredient¹** | | | 25.96 | 25.96 | 25.96 |
| **Microcrystalline cellulose (PH102)** | | | 18.00 | 12.00 | 6.00 |
| **Anhydrous lactose (21AN)** | | | 6.14 | 12.14 | 18.14 |
| **Carboxymethyl starch sodium (Type A)** | | | 8.40 | 8.40 | 8.40 |
| **Sodium stearyl fumarate (added intragranularlv)** | | | 0.3 | 0.3 | 0.3 |
| **Magnesium stearate (added intragranularlv)** | | | 0.6 | 0.6 | 0.6 |
| **Sodium stearyl fumarate (added extragranularlv)** | | | 0.6 | 0.6 | 0.6 |
| **Process of compressing large tablets (called "slugs")** | | | There was no punch sticking, and the large tablets were smooth | There was no punch sticking, and the large tablets were smooth | There was no punch sticking, and the large tablets were smooth |
| **Hardness of uncoated tablet (kgf)** | | | 6.60 | 6.67 | 6.89 |
| **Cumulative dissolution rate (%)** | **0.1 M HCl** | **15 min** | 90 | 83 | 89 |

Moreover, the hardness of uncoated tablet is also one of the factors that affect the dissolution rate. Uneven hardness may lead to uneven dissolution, thereby affecting the efficacy. Table 3 below shows the corresponding dissolution profile of the prescription of the present invention at different hardnesses. It can be seen from the table that the prescription of the present invention can eliminate the effect of hardness changes on the dissolution result of the uncoated tablet under different hardness conditions, and enable the product to maintain a good dissolution rate. Therefore, the prescription of the present invention can overcome the adverse effect caused by the different hardness of uncoated tablets.

**Table 3 Dissolution rate test of the prescription of the present invention at different hardnesses**

| | | | Proportion (%) | | | Prescription amount (g) | | |
|---|---|---|---|---|---|---|---|---|
| **Active ingredient** | | | 43.27 | | | 25.96 | | |
| **Microcrystalline cellulose** | | | 30.00 | | | 18.0 | | |
| **Anhydrous lactose** | | | 10.23 | | | 6.14 | | |
| **Carboxymethyl starch sodium** | | | 14.00 | | | 8.4 | | |
| **Sodium stearyl fumarate (added intragranularly)** | | | 0.50 | | | 0.3 | | |
| **Magnesium stearate (added intragranularly)** | | | 1.00 | | | 0.6 | | |
| **Sodium stearyl fumarate (added extragranularly)** | | | 1.00 | | | 0.6 | | |
| **Hardness range (kgf)** | | | 5.50 | | 10.30 | | 6.20 | |
| **Dissolution profile in 30 minutes** | | | Almost no accumulation at the bottom of the bottle | | Almost no accumulation at the bottom of the bottle | | Almost no accumulation at the bottom of the bottle | |
| **Cumula tive dissoluti on rate (%)** | **0.1 M hydroc hloric acid** | **n=6** | Averange | RSD | Averange | RSD | Averange | RSD |
| | | **5 min** | 41 | 13.1 | 36 | 5.9 | 60 | 5.3 |
| | | **15 min** | 88 | 2.7 | 88 | 1.4 | 91 | 3.4 |
| | | **30 min** | 90 | 1.9 | 92 | 1.2 | 93 | 2.3 |

### Regarding to the disintegrant:

In the pharmaceutical composition system of the present invention, the addition of the disintegrant can also affect the dissolution rate of the tablet. The inventor finds that the dissolution rate is good when the disintegrant is present in an amount of 1 to 30%. See Table 4 for details. Carboxymethyl starch sodium used as the disintegrant can ensure a good disintegration and preparation of the tablet, and the preferred dosage is 10% to 20%.

**Table 4 Dosage and adding method of the disintegrant**

| **Single-factor study** | | | **Dosage and adding method of the disintegrant** | | | | | |
|---|---|---|---|---|---|---|---|---|
| | | | **Carboxym ethyl starch sodium (14%) added intragranu larly** | **Carboxym ethyl starch sodium (6%) added intragranu larly** | **Carboxym ethyl starch sodium (16%) added intragranu larly** | **Carboxym ethyl starch sodium (8%) added intragranu larly** | **Carboxym ethyl starch sodium (16%) added intragranu larly: added extragranu larly = 1:1** | **Carboxym ethyl starch sodium (14%) added intragranu larly: added extragranu larly = 1:1** |
| **Batch** | | | 200 Tablets/g | 200 Tablets/g | 200 Tablets/g | 200 Tablets/g | 200 Tablets/g | 200 Tablets/g |
| **Active ingredient** | | | 25.96 | 25.96 | 25.96 | 25.96 | 25.96 | 25.96 |
| **Microcrystalline cellulose (PH102)** | | | 18.0 | 21.6 | 17.1 | 19.8 | 17.1 | 18.0 |
| **Anhydrous lactose (21AN)** | | | 6.14 | 7.34 | 5.84 | 6.74 | 5.84 | 6.14 |
| **Carboxymethyl starch sodium, Type A (added intragranularly)** | | | 8.4 | 3.6 | 9.6 | 6.0 | 4.8 | 4.2 |
| **Sodium stearyl fumarate (added intragranularly)** | | | 0.3 | 0.3 | 0.3 | 0.3 | 0.3 | 0.3 |
| **Magnesium stearate (added intragranularly)** | | | 0.6 | 0.6 | 0.6 | 0.6 | 0.6 | 0.6 |
| **Carboxymethyl starch sodium, Type A (added extragranularly)** | | | / | / | / | / | 4.8 | 4.2 |
| **Sodium stearyl fumarate (added extragranularly)** | | | 0.6 | 0.6 | 0.6 | 0.6 | 0.6 | 0.6 |
| **Hardness of uncoated tablet (kgf)** | | | 6.60 | 6.53 | 6.53 | 6.53 | 6.44 | 6.37 |
| **Cumu lative dissol ution rate (%)** | **0.1 M HCl** | **15 min** | 90 | 74 | 88 | 72 | 86 | 82 |

### Example 1

A pharmaceutical composition comprising the mesylate salt of N-(5-((4-(1-cyclopropyl-1H-indol-3-yl)pyrimidin-2-yl)amino)-2-((2-(dimethylamino)et hyl)(methyl)amino)-4-methoxyphenyl)acrylamide comprises the components of: 64.9 mg of the active ingredient (calculated by C₃₀H₃₅N₇O₂·CH₃SO₃H), 45.0 mg of microcrystalline cellulose (KG802), 15.3 mg of anhydrous lactose (21AN), 21.0 mg of carboxymethyl starch sodium (Type A), 0.8 mg of intragranular sodium stearyl fumarate, 1.5 mg of extragranular sodium stearyl fumarate and 1.5 mg of magnesium stearate. The prescription is as follows:

| | |
|---|---|
| Mesylate salt of the compound of formula I | 43.3% |
| Microcrystalline cellulose | 30.0% |
| Anhydrous lactose | 10.2% |
| Carboxymethyl starch sodium | 14% |
| Sodium stearyl fumarate | 0.5%+1.0% |
| Magnesium stearate | 1.0% |

The immediate-release film-coated tablets comprising the above components were prepared by the following preparation method:
(1) Pre-treatment of raw materials

| **Material name** | **Treatment method** |
|---|---|
| Microcrystalline cellulose (KG802) | Sieved with a 60-mesh sieve |
| Anhydrous lactose (21AN) | Sieved with a 60-mesh sieve |
| Carboxymethyl starch sodium (Type A) | Sieved with a 80-mesh sieve |
| Sodium stearyl fumarate | Sieved with a 80-mesh sieve |

(2) Weighing
The raw materials were weighed according to the schedule list.
(3) Mixing
1) Premixing I: the intragranular raw materials were mixed in a hopper mixer (speed: 15 rpm, time: 10 min). 2) Milling: sieve size: 1.0 mm, milling speed: 350 to 800 rpm. 3) Premixing II: the milled material and magnesium stearate were mixed in a hopper mixer (speed: 15 rpm, time: 10 min).
(4) Dry granulation
Delivery frequency conversion (Hz): 7 to 13; tableting frequency conversion (Hz): 20 to 40; granulation frequency conversion (Hz): 30 to 50; granulation sieve size: 1.0 mm; powder mesh number: 60 mesh.
(5) Total mixing
The fine granules obtained by dry granulation and a prescription amount of extragranular sodium stearyl fumarate were mixed in a hopper mixer for 10 min at a speed of 15 rpm.
(6) Tableting
The theoretical tablet weight was calculated according to the particle content. The mixture was compressed into tablets after adjusting the tablet weight and hardness.
(7) Coating
1) Formulation of a coating liquid: a coating liquid with a solid content of 10% was formulated with a film-coating premix (gastric-soluble type) and a prescription amount of purified water. The coating liquid was stirred for 60 minutes, and filtered with a 100-mesh sieve for later use.
2) Coating: the corresponding parameters were set according to the process requirements. During the coating process, the bed temperature was controlled at 30 to 40°C, and the coating weight gain was 2.0 to 4.0%.

(8) Packaging.

### Example 2

A pharmaceutical composition comprising the mesylate salt of N-(5-((4-(1-cyclopropyl-1H-indol-3-yl)pyrimidin-2-yl)amino)-2-((2-(dimethylamino)et hyl)(methyl)amino)-4-methoxyphenyl)acrylamide comprises the components of: 129.8 mg of the active ingredient (calculated by C₃₀H₃₅N₇O₂·CH₃SO₃H), 90.0 mg of microcrystalline cellulose (KG802), 30.6 mg of anhydrous lactose (21AN), 42.0 mg of carboxymethyl starch sodium (Type A), 1.6 mg of intragranular sodium stearyl fumarate, 3.0 mg of extragranular sodium stearyl fumarate and 3.0 mg of magnesium stearate. The prescription is as follows:

| | |
|---|---|
| Mesylate salt of the compound of formula I | 43.3% |
| Microcrystalline cellulose | 30.0% |
| Anhydrous lactose | 10.2% |
| Carboxymethyl starch sodium | 14% |
| Sodium stearyl fumarate | 0.5%+1.0% |
| Magnesium stearate | 1.0% |

The immediate-release film-coated tablets comprising the above components were prepared by the following preparation method:
(1) Pre-treatment of raw materials

| **Material name** | **Treatment method** |
|---|---|
| Microcrystalline cellulose (KG802) | Sieved with a 60-mesh sieve |
| Anhydrous lactose (21AN) | Sieved with a 60-mesh sieve |
| Carboxymethyl starch sodium (Type A) | Sieved with a 80-mesh sieve |
| Sodium stearyl fumarate | Sieved with a 80-mesh sieve |

(2) Weighing
The raw materials were weighed according to the schedule list.
(3) Mixing
1) Premixing I: the intragranular raw materials (microcrystalline cellulose (KG802), anhydrous lactose (21AN), carboxymethyl starch sodium (Type A), sodium stearyl fumarate, the active pharmaceutical ingredient) were mixed in a hopper mixer (speed: 15 rpm, time: 20 min). 2) Premixing II: after premixing I was completed, magnesium stearate (intragranular) was added to the hopper mixer for mixing (speed: 15 rpm, time: 10 min).
(4) Dry granulation
HFS speed: 30 to 45 rpm, VFS speed: 200 to 240 rpm, pressure wheel speed: 3 to 7 rpm, pressure wheel pressure: 20 to 25 KN, crushing knife speed: 1000 to 1500 rpm, pressure wheel gap: 1.0 mm, granulation sieve size: 1.6 mm.
(5) Total mixing
The fine granules obtained by dry granulation and a prescription amount of extragranular sodium stearyl fumarate were mixed in a hopper mixer for 10 min at a speed of 15 rpm.
(6) Tableting
The theoretical tablet weight was calculated according to the particle content. The mixture was compressed into tablets after adjusting the tablet weight and hardness.
(7) Coating
1) Formulation of a coating liquid: a coating liquid with a solid content of 10% was formulated with a film-coating premix (gastric-soluble type) and a prescription amount of purified water. The coating liquid was stirred for 60 minutes, and filtered with an 80-mesh sieve for later use.
2) Coating: the corresponding parameters were set according to the process requirements. During the coating process, the bed temperature was controlled at 30 to 40°C, and the coating weight gain was 2.0 to 4.0%.

(8) Packaging.

### Example 3

A pharmaceutical composition comprising the mesylate salt of N-(5-((4-(1-cyclopropyl-1H-indol-3-yl)pyrimidin-2-yl)amino)-2-((2-(dimethylamino)et hyl)(methyl)amino)-4-methoxyphenyl)acrylamide comprises the components of: 5.9 mg of the active ingredient (calculated by C₃₀H₃₅N₇O₂·CH₃SO₃H), 25.0 mg of microcrystalline cellulose, 54.7 mg of anhydrous lactose, 15.0 mg of carboxymethyl starch sodium, 0.5 mg of intragranular sodium stearyl fumarate, 0.9 mg of extragranular sodium stearyl fumarate and 0.5 mg of magnesium stearate. The prescription is as follows:

| | |
|---|---|
| Mesylate salt of the compound of formula I | 5.8% |
| Microcrystalline cellulose | 24.4% |
| Anhydrous lactose | 53.4% |
| Carboxymethyl starch sodium | 14.6% |
| Sodium stearyl fumarate | 0.5%+0.8% |
| Magnesium stearate | 0.5% |

The immediate-release film-coated tablets comprising the above components were prepared by the preparation method of Example 1.

### Example 4

A pharmaceutical composition comprising the mesylate salt of N-(5-((4-(1-cyclopropyl-1H-indol-3-yl)pyrimidin-2-yl)amino)-2-((2-(dimethylamino)et hyl)(methyl)amino)-4-methoxyphenyl)acrylamide comprises the components of: 31.8 mg of the active ingredient (calculated by C₃₀H₃₅N₇O₂·CH₃SO₃H), 30.0 mg of microcrystalline cellulose, 89.4 mg of anhydrous lactose, 30.0 mg of carboxymethyl starch sodium, 1.0 mg of intragranular sodium stearyl fumarate, 1.8 mg of extragranular sodium stearyl fumarate and 1.0 mg of magnesium stearate.

The immediate-release film-coated tablets comprising the above components were prepared by the preparation method of Example 2.

| | |
|---|---|
| Mesylate salt of the compound of formula I | 17.2% |
| Microcrystalline cellulose | 16.2% |
| Anhydrous lactose | 48.3% |
| Carboxymethyl starch sodium | 16.2% |
| Sodium stearyl fumarate | 0.5%+1.0% |
| Magnesium stearate | 0.5% |

### Example 5

A pharmaceutical composition comprising the mesylate salt of N-(5-((4-(1-cyclopropyl-1H-indol-3-yl)pyrimidin-2-yl)amino)-2-((2-(dimethylamino)et hyl)(methyl)amino)-4-methoxyphenyl)acrylamide comprises the components of: 47.2 mg of the active ingredient (calculated by C₃₀H₃₅N₇O₂·CH₃SO₃H), 30.0 mg of microcrystalline cellulose, 93 mg of anhydrous lactose, 30.0 mg of carboxymethyl starch sodium, 1.0 mg of intragranular sodium stearyl fumarate, 1.8 mg of extragranular sodium stearyl fumarate and 2.0 mg of magnesium stearate.

| | |
|---|---|
| Mesylate salt of the compound of formula I | 23.0% |
| Microcrystalline cellulose | 14.6% |
| Anhydrous lactose | 45.4% |
| Carboxymethyl starch sodium | 14.6% |
| Sodium stearyl fumarate | 0.5%+0.9% |
| Magnesium stearate | 1.0% |

The immediate-release film-coated tablets comprising the above components were prepared by the preparation method of Example 1.

### Example 6

A pharmaceutical composition comprising the mesylate salt of N-(5-((4-(1-cyclopropyl-1H-indol-3-yl)pyrimidin-2-yl)amino)-2-((2-(dimethylamino)et hyl)(methyl)amino)-4-methoxyphenyl)acrylamide comprises the components of: 97.35 g of the active ingredient (calculated by C₃₀H₃₅N₇O₂·CH₃SO₃H), 67.5 g of microcrystalline cellulose, 22.95 g of anhydrous lactose, 31.5 g of carboxymethyl starch sodium, 1.2 g of intragranular sodium stearyl fumarate, 2.25 g of extragranular sodium stearyl fumarate and 2.25 g of magnesium stearate.

| | |
|---|---|
| Mesylate salt of the compound of formula I | 43.3% |
| Microcrystalline cellulose | 30.0% |
| Anhydrous lactose | 10.2% |
| Carboxymethyl starch sodium | 14.0% |
| Sodium stearyl fumarate | 0.5%+1.0% |
| Magnesium stearate | 1.0% |

The immediate-release film-coated tablets comprising the above components were prepared by the preparation method of Example 2.

### Example 7

A pharmaceutical composition comprising the mesylate salt of N-(5-((4-(1-cyclopropyl-1H-indol-3-yl)pyrimidin-2-yl)amino)-2-((2-(dimethylamino)et hyl)(methyl)amino)-4-methoxyphenyl)acrylamide comprises the components of: 259.6 g of the active ingredient (calculated by C₃₀H₃₅N₇O₂·CH₃SO₃H), 180.0 g of microcrystalline cellulose, 61.2 g of anhydrous lactose, 84.0 g of carboxymethyl starch sodium, 3.2 g of intragranular sodium stearyl fumarate, 6.0 g of extragranular sodium stearyl fumarate and 6.0 g of magnesium stearate.

| | |
|---|---|
| Mesylate salt of the compound of formula I | 43.3% |
| Microcrystalline cellulose | 30.0% |
| Anhydrous lactose | 10.2% |
| Carboxymethyl starch sodium | 14.0% |
| Sodium stearyl fumarate | 0.5%+1.0% |
| Magnesium stearate | 1.0% |

The immediate-release film-coated tablets comprising the above components were prepared by the preparation method of Example 1.

### Example 8

A pharmaceutical composition comprising the mesylate salt of N-(5-((4-(1-cyclopropyl-1H-indol-3-yl)pyrimidin-2-yl)amino)-2-((2-(dimethylamino)et hyl)(methyl)amino)-4-methoxyphenyl)acrylamide comprises the components of: 97.35 g of the active ingredient (calculated by C₃₀H₃₅N₇O₂·CH₃SO₃H), 67.5 g of microcrystalline cellulose, 22.95 g of anhydrous lactose, 31.5 g of carboxymethyl starch sodium, 1.2 g of intragranular sodium stearyl fumarate, 2.25 g of extragranular sodium stearyl fumarate and 2.25 g of magnesium stearate.

| | |
|---|---|
| Mesylate salt of the compound of formula I | 43.3% |
| Microcrystalline cellulose | 30.0% |
| Anhydrous lactose | 10.2% |
| Carboxymethyl starch sodium | 14.0% |
| Sodium stearyl fumarate | 0.5%+1.0% |
| Magnesium stearate | 1.0% |

The immediate-release film-coated tablets comprising the above components were prepared by the preparation method of Example 2.

### Example 9

A pharmaceutical composition comprising the mesylate salt of N-(5-((4-(1-cyclopropyl-1H-indol-3-yl)pyrimidin-2-yl)amino)-2-((2-(dimethylamino)et hyl)(methyl)amino)-4-methoxyphenyl)acrylamide comprises the components of: 47.2 mg of the active ingredient (calculated by C₃₀H₃₅N₇O₂·CH₃SO₃H), 30.0 mg of sorbitol, 93 mg of calcium hydrophosphate, 30.0 mg of crospovidone XL, 1.0 mg of intragranular sodium stearyl fumarate, 1.8 mg of extragranular stearic acid and 2.0 mg of talc.

| | |
|---|---|
| Mesylate salt of the compound of formula I | 23.0% |
| Sorbitol | 14.6% |
| Calcium hydrophosphate | 45.4% |
| Crospovidone | 14.6% |
| Sodium stearyl fumarate | 0.5% |
| Stearic acid | 0.9% |
| Talc | 1.0% |

The immediate-release film-coated tablets comprising the above components were prepared by the preparation method of Example 1.

### Example 10

A pharmaceutical composition comprising the mesylate salt of N-(5-((4-(1-cyclopropyl-1H-indol-3-yl)pyrimidin-2-yl)amino)-2-((2-(dimethylamino)et hyl)(methyl)amino)-4-methoxyphenyl)acrylamide comprises the components of: 47.2 mg of the active ingredient (calculated by C₃₀H₃₅N₇O₂·CH₃SO₃H), 30.0 mg of sucrose, 93 mg of calcium hydrophosphate, 30.0 mg of crospovidone XL, 1.0 mg of intragranular sodium stearyl fumarate, 1.8 mg of extragranular stearic acid and 2.0 mg of talc.

| | |
|---|---|
| Mesylate salt of the compound of formula I | 23.0% |
| Sucrose | 14.6% |
| Calcium hydrophosphate | 45.4% |
| Crospovidone | 14.6% |
| Sodium stearyl fumarate | 0.5% |
| Stearic acid | 0.9% |
| Talc | 1.0% |

The immediate-release film-coated tablets comprising the above components were prepared by the preparation method of Example 1.

### Example 11

A pharmaceutical composition comprising the mesylate salt of N-(5-((4-(1-cyclopropyl-1H-indol-3-yl)pyrimidin-2-yl)amino)-2-((2-(dimethylamino)et hyl)(methyl)amino)-4-methoxyphenyl)acrylamide comprises the components of: 97.35 g of the active ingredient (calculated by C₃₀H₃₅N₇O₂·CH₃SO₃H), 67.5 g of microcrystalline cellulose, 22.95 g of maltose, 31.5 g of carboxymethyl starch sodium, 1.2 g of intragranular sodium stearyl fumarate, 2.25 g of extragranular sodium stearyl fumarate and 2.25 g of magnesium stearate.

| | |
|---|---|
| Mesylate salt of the compound of formula I | 43.3% |
| Microcrystalline cellulose | 30.0% |
| Maltose | 10.2% |
| Carboxymethyl starch sodium | 14.0% |
| Sodium stearyl fumarate | 0.5%+1.0% |
| Magnesium stearate | 1.0% |

The immediate-release film-coated tablets comprising the above components were prepared by the preparation method of Example 2.

### Example 12

A pharmaceutical composition comprising the mesylate salt of N-(5-((4-(1-cyclopropyl-1H-indol-3-yl)pyrimidin-2-yl)amino)-2-((2-(dimethylamino)et hyl)(methyl)amino)-4-methoxyphenyl)acrylamide comprises the components of: 64.9 mg of the active ingredient (calculated by C₃₀H₃₅N₇O₂·CH₃SO₃H), 45.0 mg of microcrystalline cellulose (KG802), 15.3 mg of corn starch, 21.0 mg of carboxymethyl starch sodium (Type A), 0.8 mg of intragranular sodium stearyl fumarate, 1.5 mg of extragranular sodium stearyl fumarate and 1.5 mg of magnesium stearate. The prescription is as follows:

| | |
|---|---|
| Mesylate salt of the compound of formula I | 43.3% |
| Microcrystalline cellulose | 30.0% |
| Corn starch | 10.2% |
| Carboxymethyl starch sodium | 14% |
| Sodium stearyl fumarate | 0.5%+1.0% |
| Magnesium stearate | 1.0% |

The immediate-release film-coated tablets comprising the above components were prepared by the preparation method of Example 1.

### Stability test and result analysis

### 1. Stability of the product in different dissolution media

### Chromatography condition:

**Instruments and reagents** High performance liquid chromatograph, electronic analytical balance, potassium dihydrogen phosphate (chromatographically pure), acetonitrile (chromatographically pure), reference substance for system suitability of related substance

**Chromatography condition** Octadecylsilane bonded silica gel as the filler (Waters XBridge C18, 4.6mm×150mm, 3.5 µm or a column with equivalent performance); flow rate: 1.0 ml/min; detection wavelength: 220 nm; column temperature: 35°C; injection volume: 10 µl.

**Mobile phase A** 2.72 g of potassium dihydrogen phosphate was dissolved in about 900 ml of water, the pH was adjusted to 6.0 with sodium hydroxide solution, water was added until the volume reached 1000 ml, and then the solution was mixed well, filtrated and degased.

**Mobile Phase B** Acetonitrile

**Gradient elution was carried out according to the following table** (the amount of mobile phase is scalable)

| | | | | | | |
|---|---|---|---|---|---|---|
| Time (min) | 0 | 20 | 30 | 40 | 42 | 50 |
| Mobile phase A (%) | 80 | 60 | 30 | 30 | 80 | 80 |
| Mobile phase B (%) | 20 | 40 | 70 | 70 | 20 | 20 |

**0.05% phosphoric acid solution** 0.5 ml of phosphoric acid was added to 1000 ml of water and mixed well.

**Diluent** 800 ml of 0.05% phosphoric acid solution and 200 ml of acetonitrile were mixed well.

**Table 5 Results of the stability study of the product in different media**

| **Dissolution medium** | **Time (hour)** | **0 hour** | **1 hour** | **2 hours** | **4 hours** | **8 hours** | **24 hours** |
|---|---|---|---|---|---|---|---|
| **0.1 mol/L hydrochloric acid solution** | Peak area | 5817.502 | 5724.833 | 5715.132 | 5709.908 | 5691.358 | 5709.127 |
| | Relative content | 100.0% | 98.4% | 98.2% | 98.2% | 97.8% | 98.1% |
| **Acetate buffer, pH 4.5** | Peak area | 5388.433 | 5313.248 | 5301.212 | 5294.096 | 5282.456 | 5308.160 |
| | Relative content | 100.0% | 98.6% | 98.4% | 98.2% | 98.0% | 98.5% |
| **Phosphate buffer, pH 6.8** | Peak area | 3364.067 | 3310.739 | 3294.386 | 3270.577 | 3264.820 | 3236.565 |
| | Relative content | 100.0% | 98.4% | 97.9% | 97.2% | 97.0% | 96.2% |
| **Phosphate buffer, pH 6.8 (containing 0.1% SDS)** | Peak area | 4868.623 | 4864.179 | 4861.332 | 4865.548 | 4853.247 | 4837.464 |
| | Relative content | 100.0% | 99.9% | 99.9% | 99.9% | 99.7% | 99.4% |
| **Water** | Peak area | 3627.570 | 3623.876 | 3610.467 | 3602.525 | 3592.348 | 3590.479 |
| | Relative content | 100.0% | 99.9% | 99.5% | 99.3% | 99.0% | 99.0% |
| **Water ( containing 0.1% SDS)** | Peak area | 5456.676 | 5443.102 | 5433.346 | 5430.342 | 5421.376 | 5422.712 |
| | Relative content | 100.0% | 99.8% | 99.6% | 99.5% | 99.4% | 99.4% |

The inventor studied the solution stability of the tablet prepared in Example 1 as example in 0.1 mol/L hydrochloric acid solution, acetate buffer (pH 4.5), phosphate buffer (pH 6.8), phosphate buffer containing 0.1% SDS (pH 6.8), water and water containing 0.1% SDS. According to the dissolution and release rate test (the second method described in general rule 0931 of volume IV of the Chinese Pharmacopoeia 2015 Edition) under a condition of 50 rpm, an eluate was taken at 30 minutes, and then filtered. The filtrate was used as the test solution to study the solution stability at room temperature within 24 hours. The solution stability data is shown in Table 4.

The results showed that the tablet prepared in the present invention was stable within 24 hours in 0.1 mol/L hydrochloric acid solution, acetate buffer (pH 4.5), phosphate buffer containing 0.1% SDS (pH 6.8), water and water containing 0.1% SDS; and the tablet degraded slightly in phosphate buffer (pH 6.8).

### 2. Stability under light, high temperature or high humidity conditions

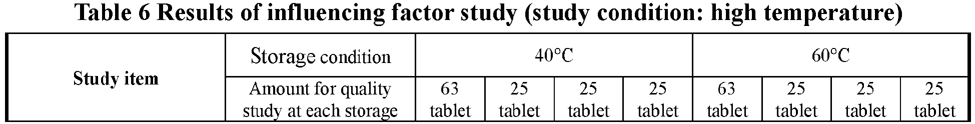

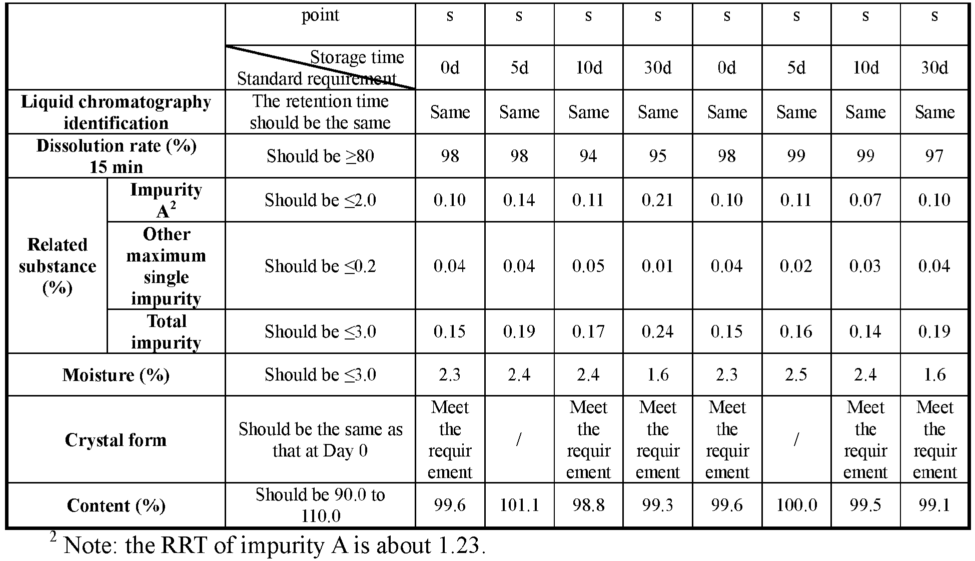

The structure of impurity A is as follows:

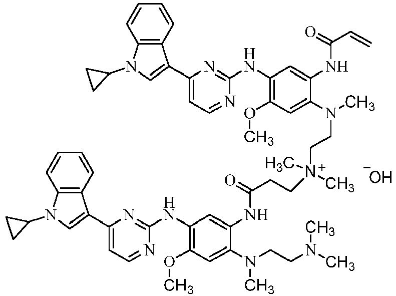

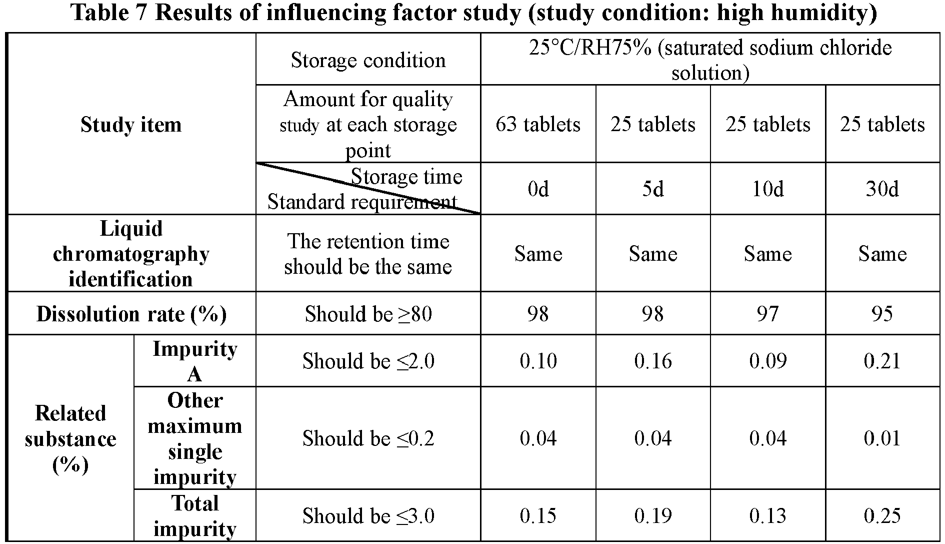

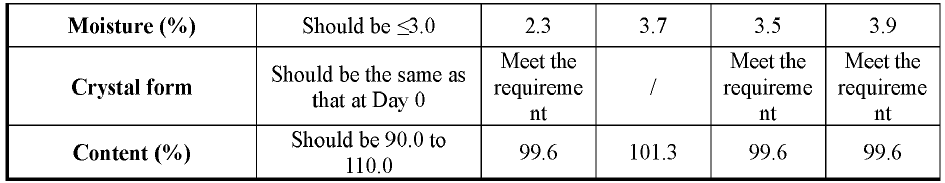

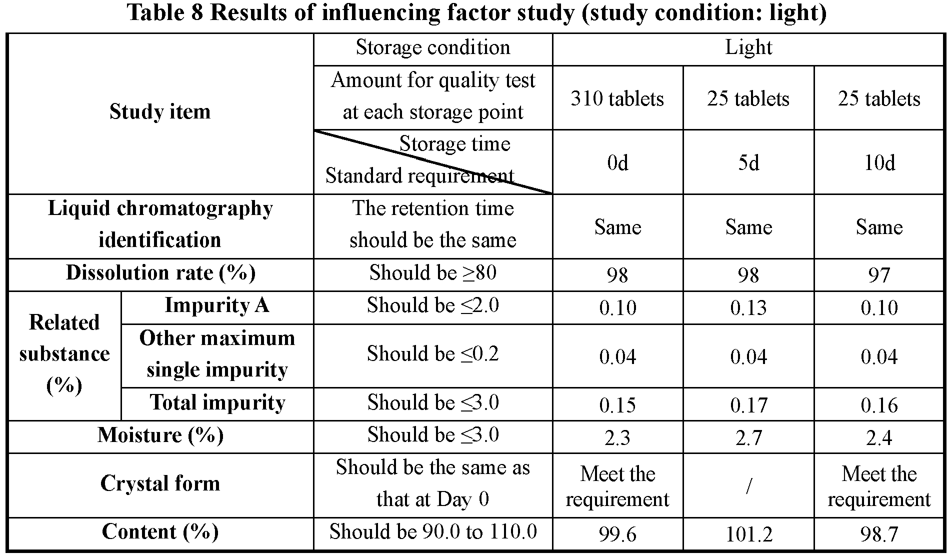

The results are shown in Tables 6 to 8. The influencing factor study was conducted for 30 days (the study under light condition was conducted for 10 days, the total illumination was not less than 1.2×106 lux·hr). The results showed that the product was stable under light condition (total illuminance 1.2×106 lux/hr) without degradation, and there was no obvious change in various quality indicators. Under high temperature (40°C), high temperature (60°C) and 25°C/RH75% conditions, the related substance slightly degraded, but there was no obvious change in other quality indicators, and the sample had a good stability.

### 3. Product stability

**Table 9 Results of product quality study in the stability test (product of Example 1)**

| **Study item** | | **Limit requirement** | **Time** | | | |
|---|---|---|---|---|---|---|
| | | | **0 day** | **1 month** | **2 months** | **3 months** |
| **Appearance** | | **A light yellow film-coated tablet, being off-white to light yellow after removing the coating** | A Light yellow film -coated tablet, being off-white after removing the coating | A Light yellow film -coated tablet, being off-white after removing the coating | A Light yellow film-coated tablet, being off-white after removing the coating | A Light yellow film-coated tablet, being off-white after removing the coating |
| **Dissolution rate (%)** | | **Should be ≥80** | 97 | 98 | 96 | 99 |
| **Related substance (%)** | **Impurity A** | **Should be ≤2.0** | 0.11 | 0.17 | 0.19 | 0.18 |
| | **Other maximum single impurity** | **Should be ≤0.2** | 0.08 | 0.06 | 0.06 | 0.06 |
| | **Total impurity** | **Should be ≤53.0** | 0.44 | 0.38 | 0.45 | 0.46 |
| **Content (%)** | | **Should be 90.0 to 110.0** | 98.6 | 100.1 | 98.8 | 99.0 |

**Table 10 Results of product quality study in the stability test (product of Example 2)**

| **Study item** | | **Limit requirement** | **Time** | | | |
|---|---|---|---|---|---|---|
| | | | **0 day** | **1 month** | **2 month** | **3 month** |
| **Appearance** | | **A pink film-coated tablet, being off-white to light yellow after removing the coating** | A pink film -coated tablet, being off-white after removing the coating | A pink film -coated tablet, being off-white after removing the coating | A pink film-coated tablet, being off-white after removing the coating | A pink film-coated tablet, being off-white after removing the coating |
| **Dissolution rate (%)** | | **Should be ≥80** | 98 | 99 | 97 | 99 |
| **Related substance (%)** | **Impurity A** | **Should be ≤2.0** | 0.10 | 0.18 | 0.27 | 0.21 |
| | **Other maximum single impurity** | **Should be ≤0.2** | 0.04 | 0.02 | 0.01 | 0.01 |
| | **Total impurity** | **Should be ≤3.0** | 0.15 | 0.22 | 0.29 | 0.25 |
| **Content (%)** | | **Should be 90.0 to 110.0** | 99.6 | 99.7 | 100.9 | 100.6 |

It can be seen from Tables 9 and 10 that the tablet prepared in the present invention had a good quality, the product quality was stable in the stability test. The prescription and process of the product were stable, and the reproducibility was good.

The samples prepared in other Examples all had similar stability results to those of the samples of Examples 1 and 2.

### Dissolution test and result analysis

1. Four batches of the product prepared in Example 1 were randomly selected for testing. The results are shown in Table 10.

**Table 11 Dissolution curve of the product in 0.1 M hydrochloric acid medium (product of Example 1)**

| **Product of Example 1** | | **Sample 1** | **Sample 2** | **Sample 3** | **Sample 4** |
|---|---|---|---|---|---|
| **Cumulative dissolution rate (%)** | **5 min** | 51 | 47 | 57 | 76 |
| | **10 min** | 94 | 88 | 97 | 97 |
| | **15 min** | 98 | 94 | 99 | 100 |
| | **30 min** | 99 | 97 | 100 | 101 |
| | **45 min** | 100 | 98 | 100 | 102 |

The results showed that the obtained samples all had good dissolution uniformity.

2. The dissolution test of the product prepared in Example 2 in different media was carried out. The results are shown in Table 12.

**Table 12 Dissolution data of the product in different media (product of Example 2)**

| **Dissolution medium** | | **0.1 mol/L hydrochloric acid** | **Buffer, pH 4.5** | **Buffer containing 0.1% SDS, pH 6.8** | **Water containing 0.1% SDS** |
|---|---|---|---|---|---|
| **Cumulative dissolution rate (%)** | **5 min** | 34 | 30 | 66 | 41 |
| | **10 min** | 84 | 76 | 86 | 75 |
| | **15 min** | 97 | 92 | 90 | 87 |
| | **30 min** | 101 | 96 | 91 | 93 |
| | **45 min** | 101 | 96 | 92 | 90 |
| | **60 min** | 101 | 97 | 92 | 94 |

The results showed that the product prepared in the present invention had good dissolution rate in different media.

The samples prepared in other Examples had similar dissolution profile to those of the samples of Examples 1 and 2.

## Claims

1. A pharmaceutical composition, comprising N-(5-((4-(1-cyclopropyl-1H-indol-3-yl)pyrimidin-2-yl)amino)-2-((2-(dimethylamino)et hyl)(methyl)amino)-4-methoxyphenyl)acrylamide, an isomer, solvate, hydrate, pharmaceutically acceptable salt thereof or a combination thereof as the active ingredient, and at least one pharmaceutically acceptable excipient.

2. The pharmaceutical composition according to claim 1, **characterized in that** the active ingredient is a mesylate salt of N-(5-((4-(1-cyclopropyl-1H-indol-3-yl)pyrimidin-2-yl)amino)-2-((2-(dimethylamino)et hyl)(methyl)amino)-4-methoxyphenyl)acrylamide.

3. The pharmaceutical composition according to claim 1 or 2, **characterized in that** the active ingredient is present in an amount of 1 to 60%, and preferably 35 to 50%.

4. The pharmaceutical composition according to claim 3, **characterized in that** the unit dose of the active ingredient is 10 to 200 mg, preferably 55 to 110 mg, and more preferably 55 mg and 110 mg.

5. The pharmaceutical composition according to claim 1 or 2, **characterized in that** the excipient comprises one or more filler(s), comprising at least one disaccharide or polysaccharide, such as glucan, starch, cellulose, lactose, maltose or sucrose.

6. The pharmaceutical composition according to claim 5, **characterized in that** the disaccharide or polysaccharide is present in an amount of 1 to 60%, preferably 5 to 55%, more preferably 5 to 30%, and further preferably 5 to 15%; the preferred filler is a disaccharide, such as lactose.

7. The pharmaceutical composition according to claim 5, **characterized in that** the filler also comprises one or more of microcrystalline cellulose, mannitol, sorbitol, calcium hydrophosphate and calcium sulfate, and preferably microcrystalline cellulose.

8. The pharmaceutical composition according to claim 5, **characterized in that** the filler is selected from the group consisting of microcrystalline cellulose and lactose.

9. The pharmaceutical composition according to claim 8, **characterized in that** the microcrystalline cellulose is present in an amount of 1 to 60%, preferably 10 to 40%, and more preferably 20 to 40%; and the lactose is present in an amount of 1 to 60%, preferably 5 to 30%, and more preferably 5 to 15%.

10. The pharmaceutical composition according to claim 8, **characterized in that** the weight ratio of microcrystalline cellulose to lactose is 1:3 to 3:1, and preferably 2-3:1.

11. The pharmaceutical composition according to claim 5, **characterized in that** the filler is selected from the group consisting of microcrystalline cellulose and anhydrous lactose.

12. The pharmaceutical composition according to claim 11, **characterized in that** the microcrystalline cellulose is present in an amount of 1 to 60%, preferably 10 to 40%, and more preferably 20 to 40%; and the anhydrous lactose is present in an amount of 1 to 60%, preferably 5 to 30%, and more preferably 5 to 15%.

13. The pharmaceutical composition according to claim 11, **characterized in that** the weight ratio of microcrystalline cellulose to anhydrous lactose is 1:3 to 3:1, and preferably 2-3:1.

14. The pharmaceutical composition according to claim 5, **characterized in that** the filler is present in an amount of 20 to 80%, and preferably 30 to 50%.

15. The pharmaceutical composition according to claim 1 or 2, **characterized in that** the excipient comprises one or more disintegrant(s).

16. The pharmaceutical composition according to claim 15, **characterized in that** the disintegrant is one or more selected from the group consisting of low-substituted hydroxypropyl cellulose, croscarmellose sodium, carboxymethyl starch sodium and crospovidone, and preferably carboxymethyl starch sodium.

17. The pharmaceutical composition according to claim 15, **characterized in that** the disintegrant is present in an amount of 1 to 30%, and preferably 10 to 20%.

18. The pharmaceutical composition according to claim 15, **characterized in that** the disintegrant is added intragranularly.

19. The pharmaceutical composition according to claim 1 or 2, **characterized in that** the excipient comprises one or more lubricant(s).

20. The pharmaceutical composition according to claim 19, **characterized in that** the lubricant is one or more selected from the group consisting of talc, stearic acid, sodium stearyl fumarate, glyceryl behenate, magnesium stearate and micronized silica gel, and preferably sodium stearyl fumarate and magnesium stearate.

21. The pharmaceutical composition according to claim 19, **characterized in that** the lubricant is present in an amount of 0.1 to 10%, and preferably 0.2 to 5%.

22. The pharmaceutical composition according to claim 19, **characterized in that** the lubricant is selected from the group consisting of sodium stearyl fumarate and magnesium stearate, the sodium stearyl fumarate is present in an amount of 0.1 to 5%, and preferably 0.3 to 3%, and the magnesium stearate is present in an amount of 0.1 to 5%, and preferably 0.2 to 2%.

23. The pharmaceutical composition according to claim 1 or 2, **characterized by** comprising the following components:
| | |
|---|---|
| active ingredient | 35 to 50% |
| lactose | 5 to 15% |
| microcrystalline cellulose | 30 to 50% |
| carboxymethyl starch sodium | 10 to 20% |
| lubricant | 0.5 to 5%. |

24. The pharmaceutical composition according to claim 23, **characterized by** comprising the following components:
| | |
|---|---|
| active ingredient | 43.3% |
| lactose | 10.2% |
| microcrystalline cellulose | 30.0% |
| carboxymethyl starch sodium | 14% |
| lubricant | 0.5 to 5% |

25. The pharmaceutical composition according to claim 1 or 2, **characterized in that** the pharmaceutical composition is an oral formulation, preferably a tablet or capsule, more preferably a coated tablet, and further preferably an immediate-release film-coated tablet.

26. The pharmaceutical composition according to claim 25, **characterized in that** the coating agent of the immediate-release film-coated tablet is a gastric-soluble film coating premix.

27. A method for preparing the pharmaceutical composition according to any one of claims 1 to 26, **characterized by** comprising the following steps of:
1) pre-treatment of raw materials: sieving the filler, disintegrant and intragranular lubricant for later use;
2) mixing: weighing the intragranular raw materials according to specified amounts and mixing them;
3) dry granulation: granulating the above mixed powder by dry granulation;
4) total mixing: mixing the resulting granules and extragranular lubricant;
5) optionally, tableting; and
6) optionally, coating.

28. The method according to claim 27, comprising the specific steps of:
1) pre-treatment of raw materials: sieving microcrystalline cellulose, lactose, sodium stearyl fumarate and carboxymethyl starch sodium for later use;
2) mixing: weighing the intragranular raw materials according to prescription amounts, and mixing microcrystalline cellulose, lactose, carboxymethyl starch sodium, sodium stearyl fumarate, the active ingredient and magnesium stearate with a hopper mixer;
3) dry granulation: granulating the above mixed powder with a dry granulator;
4) total mixing: mixing the resulting fine granules and prescription amount of extragranular sodium stearyl fumarate with a hopper mixer;
5) optionally, tableting; and
6) optionally, coating: i) formulation of a coating liquid, adding a prescription amount of Opadry to purified water under stirring to formulate a coating liquid with a solid content of 10%, stirring the coating liquid evenly, and sieving the coating liquid for later use; and ii) finishing the coating until the coating weight gain reaches about 2.0% to 4.0%.
